# EUROPEAN PATENT APPLICATION

(11) **EP 3 593 713 A1**
(43) Date of publication of application: **15.01.2020**
(21) Application number: 17900240.7
(22) Date of filing: 13.11.2017
(51) Int. Cl.: A61B 5/0408, C01B 32/00

(54) **ELECTRODE FOR BIOLOGICAL INFORMATION MEASUREMENT**

(30) Priority: 07.03.2017 JP 2017042282
(71) Applicant: Alps Alpine Co., Ltd., Ota-ku, Tokyo 1458501 (JP)
(72) Inventor: AKIYAMA, Toshinori, Tokyo 145-8501 (JP); SUZUKI, Tsuyoshi, Tokyo 145-8501 (JP)
(74) Representative: Schmitt-Nilson Schraud Waibel Wohlfrom Patentanwälte Partnerschaft mbB
(86) International application number: PCT/JP2017/040817
(87) International publication number: WO 2018/163511

(57) **Abstract**

An electrode for biological information measurement is provided as an electrode for measuring biological information, such as an electrocardiogram, a pulse wave, or an electroencephalogram. The biological information can be stably obtained with the electrode without using a flowable electrolyte. The electrode for biological information measurement includes an electrode, at least a portion of which is made of a carbon material, and a terminal electrically connected to the electrode, wherein part of the surface of the electrode made of the carbon material can come into contact with a biological body, and part of the surface is modified with a cationic functional group.

## Description

### Technical Field

The present invention relates to an electrode for biological information measurement for use in the measurement of an electroencephalogram or the like and a method for producing the electrode for biological information measurement.

### Background Art

In recent years, various types of biological information, for example, a pulse wave, an electrocardiogram, an electromyogram, a body fat, and an electroencephalogram, have been more frequently measured. For stable contact with a biological body, various electrodes for biological information measurement have been proposed. In particular, various modifications have been made to an electroencephalography electrode that is to be attached to the head with hair. For example, to bring an electrode into contact with the scalp through the hair, the electrode tip must have a small area. However, an electrode made of a hard material, such as metal, and having a tip with a small area causes pain in the user. Thus, an electrically conductive gel is sometimes used to stabilize the contact between an electroencephalography electrode and the scalp without making the electrode tip narrow.

An electroencephalogram helmet described in Patent Literature 1 as a conventional example that uses an electrically conductive gel includes a sensor component (corresponding to an electrode) equipped with a forward and backward movement means and a tubular electrode, and the tubular electrode is coupled to a paste supply means for automatically supplying an electrolyte paste to the scalp.

### Citation List

### Patent Literature

PTL 1: Japanese Unexamined Patent Application Publication No. 2004-254953

### Summary of Invention

### Technical Problem

However, the tubular electrode of the electroencephalogram helmet described in Patent Literature 1 is an extensive mechanism due to the structure for supplying an electrically conductive gel (the paste supply means). The helmet has an increased size and weight, which is a heavy burden to the measurer during measurement. Furthermore, due to an increased amount of electrolyte paste supplied to the scalp (measurement site) during measurement, removal of the paste from the scalp after the measurement is also a heavy workload.

It is an object of the present invention to provide an electrode for biological information measurement for measuring biological information, such as an electrocardiogram, a pulse wave, or an electroencephalogram, wherein the biological information can be stably obtained with the electrode for biological information measurement without using a flowable electrolyte (specific examples include liquid, paste, and gel) as described in Patent Literature 1. It is another object of the present invention to provide a method for producing the electrode for biological information measurement.

### Solution to Problem

The present invention provided to solve the above problems is in one aspect an electrode for biological information measurement that includes an electrode, at least a portion of which is made of a carbon material, and a terminal electrically connected to the electrode, wherein at least part of a surface of the portion of the electrode made of the carbon material can come into contact with a biological body, and part of the surface is modified with a cationic functional group.

The surface of the portion made of the carbon material in the electrode of the electrode for biological information measurement has a graphene structure, and the π electron cloud of the graphene structure transfers electrons. Electric signals of biological information on the surface of a biological body are mainly transmitted by chloride ions. Thus, on the surface of a portion coming into contact with a biological body in the portion made of the carbon material, charge transfer by chloride ions on the biological body is changed to charge transfer by electrons of the π electron cloud on the electrode surface, that is, there is a carrier conversion of electric charges.

In the electrode for biological information measurement according to the present invention, part of the surface of a portion coming into contact with a biological body in the portion made of the carbon material is modified with a cationic functional group. More specifically, the cationic functional group is bonded to part of carbon of the graphene structure on the surface of the portion coming into contact with a biological body. Thus, positive charges having the cationic functional group are stabilized by interaction with the π electron cloud of the graphene structure. Thus, chloride ions near the surface of the portion coming into contact with a biological body are likely to interact with positive charges of the cationic functional group on the surface. Thus, electric charges of the chloride ions can easily enter the π electron cloud of the graphene structure of the portion coming into contact with a biological body through the cationic functional group, thus resulting in lower polarization. This also decreases temporal accumulation of electric charges (an increased concentration of chloride ions near the electrode surface) and facilitates electric charge transfer between the biological body and the electrode. This enables stable measurement without using an electrolyte solution or an electrically conductive gel.

In the electrode for biological information measurement, the cationic functional group may include a cationic amino group. The surface of the carbon material can be relatively easily modified with an amino group. An amino group can be converted into a cationic functional group by the addition of a proton. Thus, the use of a cationic amino group as a cationic functional group can easily increase the density of the cationic functional group on the carbon surface and can more stably reduce the effects of chloride ions of the biological body.

In the electrode for biological information measurement, at least part of the cationic functional group has preferably formed a salt with an anion. If the cationic functional group has not formed a salt, the cationic functional group interacts with water and forms hydroxide ions, which makes the solution around the carbon material alkaline. Exchange between the hydroxide ions and chloride ions from the biological body in this state changes the pH of the solution around the carbon material. In contrast, when the cationic functional group has previously formed a salt, exchange with chloride ions from the biological body can occur with no or little pH change of the solution around the carbon material.

In an electrode for biological information measurement on which at least part of the cationic functional group has formed a salt with an anion, the anion preferably includes chloride ions. When the cationic functional group has previously formed an ion pair with a chloride ion, chloride ions interacting with the cationic functional group can be easily exchanged with chloride ions from the biological body.

Another aspect of the present invention is a method for producing an electrode for biological information measurement that includes an electrode, at least a portion of which is made of a carbon material, and a terminal electrically connected to the electrode, wherein at least part of the surface of the portion of the electrode made of the carbon material can come into contact with a biological body. The production method includes an electrolytic oxidation step of anodically electrolyzing the surface while the surface is in contact with an ammonium carbamate solution and a reduction step of bringing the surface oxidized in the electrolytic oxidation step into contact with a solution containing ammonia, sodium dithionate, and sodium carbonate to reduce the surface. The electrode for biological information measurement, the surface of which is partly modified with a cationic functional group, can be produced by the production method with less chemical damage to the electrode constituent material.

The production method may further include a chlorination step of bringing the surface reduced in the reduction step into contact with an acidic liquid containing chloride ions. The chlorination step enables the production of an electrode for biological information measurement with which biological information can be more stably obtained.

### Advantageous Effects of Invention

The present invention provides an electrode for biological information measurement with which biological information can be stably obtained without using a flowable electrolyte and a method for producing the electrode for biological information measurement.

### Brief Description of Drawings

[Fig. 1] Fig. 1 is an explanatory view of the structure of an electrode for biological information measurement (biological electrode) according to an embodiment of the present invention.
[Fig. 2] Fig. 2 is an explanatory view of the structure of a metallic biological electrode according to the related art.
[Fig. 3] Fig. 3(a) is a graph of data obtained by electroencephalography with the biological electrode illustrated in Fig. 2 and data obtained by low-cut filtering of the data, and Fig. 3(b) is a graph of data obtained by electroencephalography with a biological electrode according to a comparative example and data obtained by low-cut filtering of the data.
[Fig. 4] Fig. 4(a) is a graph of the data subjected to low-cut filtering illustrated in Fig. 3(a) with a different vertical axis, and Fig. 4(b) is a graph of the data subjected to low-cut filtering illustrated in Fig. 3(b) with a different vertical axis.
[Fig. 5] Fig. 5 is a diagram of an equivalent circuit of a biological electrode.
[Fig. 6] Fig. 6 is a schematic view of charge transfer caused by contact between a biological electrode according to an embodiment of the present invention and a biological body.
[Fig. 7] Fig. 7 is a graph of a result of X-ray photoelectron spectroscopy (XPS) measurement of the surface of a carbon fiber of a biological electrode according to an embodiment of the present invention.
[Fig. 8] Fig. 8 is a graph of a result of impedance measurement of a biological electrode according to another embodiment of the present invention and the biological electrode according to the comparative example immersed in 0.1 M saline.
[Fig. 9] Fig. 9(a) is a graph of data obtained by electroencephalography in which a biological electrode according to an example is fixed to the forehead of a human, and an earlobe is used as a ground point and a reference, and data obtained by low-cut filtering of the data, and Fig. 9(b) is a graph of data obtained by electroencephalography in which the biological electrode according to the comparative example is fixed to the forehead of a human, and an earlobe is used as a ground point and a reference, and data obtained by low-cut filtering of the data.
[Fig. 10] Fig. 10 is a schematic view of a process of the method of modification with a -NH₃⁺ group, which is a cationic functional group, in an ammonium carbamate solution.
[Fig. 11] Fig. 11(a) is a picture of the appearance of a carbon fiber focusing electrode, Fig. 11(b) is a picture of the appearance of a CC composite block, Fig. 11(c) is a picture of the appearance of a three-dimensional electrode, which is an example of a comparison electrode, and Fig. 11(d) is a picture of the appearance of a (gold-plated dish shaped electrode), which is another example of a comparison electrode.
[Fig. 12] Fig. 12(a) is a photograph of an electrolytic treatment apparatus (electrolyzer), and Fig. 12(b) is a schematic view of the structure of an electrolytic treatment apparatus (electrolyzer).
[Fig. 13] Fig. 13 is a result of ESCA measurement of a cross section of an electrode tip after an electrolytic oxidation step of the CC composite illustrated in Fig. 11(b) performed for different hours.
[Fig. 14] Fig. 14 is a result of impedance measurement of an untreated sample, a sample after electrolysis, and a sample after reduction in 0.1 M aqueous NaCl.
[Fig. 15] Fig. 15 is a graph of data obtained by electroencephalography with a comparison electrode.
[Fig. 16] Fig. 16 is a graph of data obtained by electroencephalography with a carbon fiber focusing electrode.

### Description of Embodiments

Embodiments of the present invention are described below with reference to the accompanying drawings.

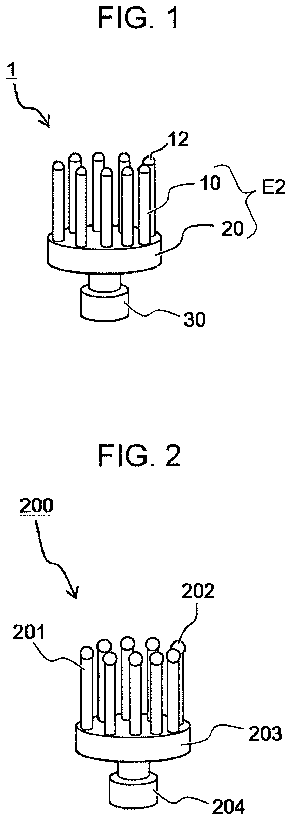

Fig. 1 is an explanatory view of the structure of an electrode for biological information measurement (hereinafter also referred to as a "biological electrode") according to an embodiment of the present invention. Fig. 2 is an explanatory view of the structure of a metallic gold electrode 200 according to the related art.

As illustrated in Fig. 1, a biological electrode 1 according to an embodiment of the present invention is composed of an electrode E2 made of a carbon material and a terminal 30 electrically connected to the electrode E2. Part of the surface of the electrode E2 in the biological electrode 1 is brought into contact with a biological body to measure biological information of the biological body.

First, the electrode E2 of the biological electrode 1 is composed of a composite material containing carbon fibers bound with a matrix of a carbon-containing material and, as illustrated in Fig. 1, is composed mainly of a plurality of pins 10 that can come into contact with a biological body and a base 20 coupled to the pins 10. At least the carbon fibers are electrically conductive, and therefore the entire electrode E2 of the biological electrode 1 is electrically conductive. The entire electrode E2 is formed of the carbon material. Although the composite material containing carbon fibers bound with the matrix of the carbon-containing material is used as a carbon material in the electrode E2 of the biological electrode 1, the electrode E2 may be formed of another material. For example, a carbon fiber reinforced carbon composite material produced by carbonization of a synthetic resin matrix or a graphite material (carbon graphite) may be used.

More specifically, as an example, the electrode E2 of the biological electrode 1 illustrated in Fig. 1 includes nine pins 10 each protruding from the base 20. The surface of the tip of each pin 10 in the protruding direction can come into direct contact with a biological body. The tip is hereinafter referred to as a biological body contact end 12. In the biological electrode 1, the biological body contact ends 12 of the electrode E2 are subjected to surface treatment as described later to modify part of the surface of each biological body contact end 12 with a cationic functional group.

In the electrode E2, a disk-shaped base 20 several centimeters in diameter is opposite to the biological body contact ends 12, and the pins 10 protrude from one surface of the base 20. The number of pins 10 is nine in Fig. 1 but may be one or two or more.

As described above, the electrode E2 of the biological electrode 1 is composed of the composite material containing carbon fibers bound with the matrix of the carbon-containing material. Specific examples of the material of the matrix include cured materials of epoxy resins and carbonized materials produced by firing synthetic resins at approximately 2000°C. Specific examples of carbon fibers include polyacrylonitrile (PAN) fibers derived from acrylic fibers and pitch fibers derived from pitch. Although the carbon fibers bound with the matrix are suitably used, a bundle of carbon fibers may also be used.

Although the pins 10 and the base 20 integrally constitute the electrode E2, the electrode E2 may have another structure. For example, only the pins 10 are made of a carbon material, and the base 20 may be made of an electrically conductive metallic material. For example, only a portion of the electrode E2 having a surface that can come into contact with a biological body (the biological body contact end 12 of the biological electrode 1) may be made of a carbon material, and the other portion may be made of an electrically conductive metallic material.

The electrode E2 of the biological electrode 1 suitable for electroencephalography has the pins 10 protruding from the base 20 and has the biological body contact end 12 at the tip of each pin 10 so that the electrode E2 can come into contact with a biological body through the hair. However, the electrode E2 may have another structure. For example, an electrode for biological information measurement suitable for electrocardiography has an electrode made of a carbon material on a surface of a flexible base such that the electrode can move smoothly along the surface of the skin (biological body). Part of the surface of the electrode comes into contact with the biological body. It goes without saying that part of the surface is modified with a cationic functional group.

Next, the terminal 30 of the biological electrode 1 is made of a metallic material, such as gold, copper, or a copper alloy, and has a function of electrical connection to an external device (measuring apparatus). As illustrated in Fig. 1, the terminal 30 is located on a surface of the base 20 of the electrode E2 opposite the surface on which the pins 10 are located. Although not shown in the figure, the terminal 30 is coupled to a lead wire and a terminal, which is coupled to a measuring apparatus. Thus, electric signals from the biological body are transmitted to the measuring apparatus.

For comparison purposes, Fig. 2 illustrates the structure of a dry-type electrode for biological information measurement according to the related art (hereinafter also referred to as a "gold electrode"). As illustrated in Fig. 2, the electrode for biological information measurement according to the related art (the gold electrode 200) has nine metallic pins 201 and a metallic sphere 202 at the tip of each pin 201. The sphere 202 can come into direct contact with a biological body. Each end of the nine pins 201 opposite the sphere 202 is joined to a disk-shaped metallic base 203 several centimeters in diameter. The pins 201 protrude from a surface of the base 203. A metallic terminal 204 to be electrically connected to an external device is located on a surface of the base 203 opposite the surface on which the pins 201 are located. The gold electrode 200 is entirely plated with gold to improve its electrical conductivity and corrosion resistance. When the gold electrode 200 is used, the thin gold electrode 200 is pressed against the scalp such that the gold electrode 200 can come into contact with the scalp through the hair. This gives the user an unpleasant feeling.

A conventional electrode for biological information measurement (hereinafter also referred to as a "biological electrode according to a comparative example") that has a structure similar to the structure of the biological electrode 1 according to an embodiment of the present invention but a portion of which corresponding to the biological body contact end 12, which is a portion coming into contact with a biological body, is not subjected to surface treatment described later tends to cause a phenomenon called an artifact, as described below. The artifact is the phenomenon that causes abrupt baseline variations that cannot be leveled even using a baseline correction filter in the measurement of an electroencephalogram with the biological electrode according to the comparative example.

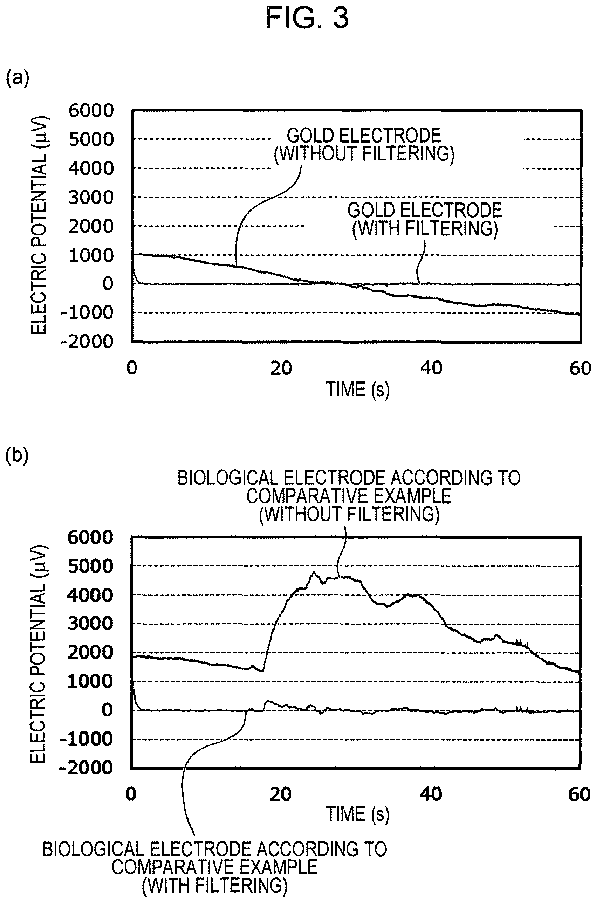

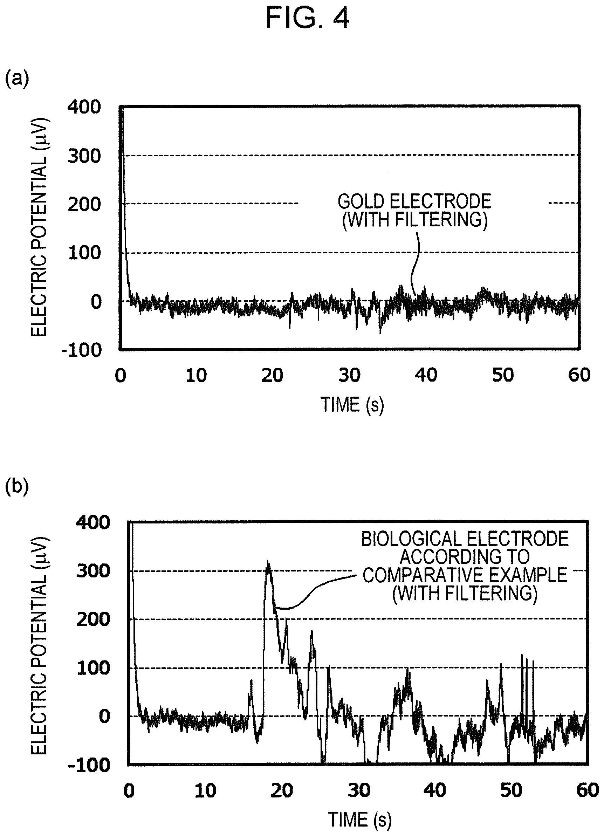

Fig. 3(a) is a graph of data obtained by electroencephalography with the gold electrode 200 according to the related art illustrated in Fig. 2 and data obtained by low-cut filtering (cutting of signals of 0.5 Hz or less; the same applies hereinafter) of the data. Fig. 3(b) is a graph of data obtained by electroencephalography with the biological electrode according to the comparative example and data obtained by low-cut filtering of the data. Fig. 4(a) is a graph of the data subjected to low-cut filtering illustrated in Fig. 3(a) with a different vertical axis, and Fig. 4(b) is a graph of the data subjected to low-cut filtering illustrated in Fig. 3(b) with a different vertical axis. In the graphs in Figs. 3 and 4, the horizontal axis represents the elapsed time (s), and the vertical axis represents the output electric potential (µV). The vertical axes of the graph in Fig. 3(a) and the graph in Fig. 3(b) have the same range (-2000 to 6000 µV), and the vertical axes of the graph in Fig. 4(a) and the graph in Fig. 4(b) have the same range (-100 to 400 µV).

As shown in Fig. 3(a), for the gold electrode 200, the baseline drifts during the measurement but does not vary abruptly. Thus, the baseline can be almost flat after baseline correction filtering. In contrast, as shown in Fig. 3(b), for the biological electrode according to the comparative example, the measured electric potential varies abruptly for approximately 20 seconds. Thus, the baseline cannot be flat even after the baseline correction filtering performed in the case of the gold electrode 200.

More specifically, in the result for the gold electrode 200 in Fig. 4(a), low-cut filtering for baseline correction gives an almost flat baseline with noises of tens of microvolts. In contrast, in the result for the biological electrode according to the comparative example in Fig. 4(b), the baseline is not flat even after low-cut filtering. On the contrary, peaks are formed as a result of failing to reduce the effects of the artifact (typical example: 20 seconds). If the peaks are considered to be signals based on an electroencephalogram, the peaks are misunderstood as an abnormal electroencephalogram.

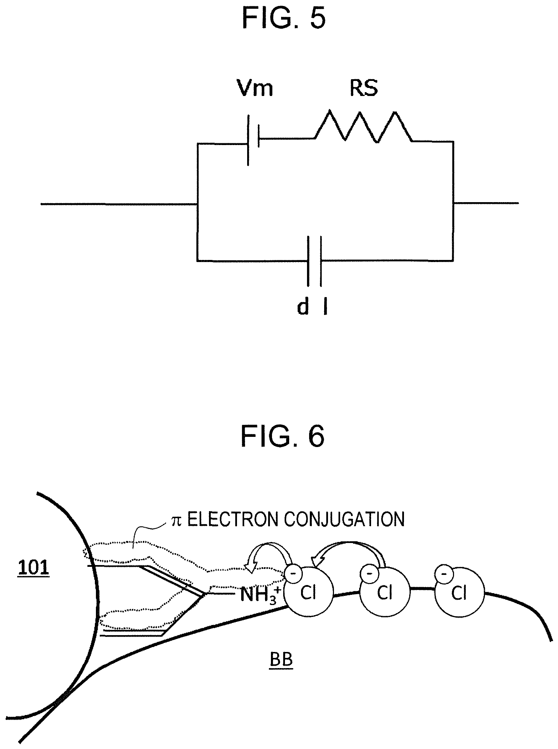

Fig. 5 illustrates an equivalent circuit of the biological electrode 1. In the equivalent circuit illustrated in Fig. 5, a polarization voltage Vm and a resistance Rs are connected in series, and the partial circuit composed of the polarization voltage Vm and the resistance Rs is parallel to a capacitance dl. The potential difference due to the polarization voltage Vm results from electric charges of the leakage current from an apparatus and the like accumulated between the biological body contact end 12 of the biological electrode 1 and a biological body. Conversion of electric charges occurs between a biological body and the biological electrode 1. Ions, particularly chloride ions, carry charges on the biological body surface, and electrons carry charges in the biological electrode 1. Thus, electric charges transferred as chloride ions are received as electrons at the interface between the biological body and the biological electrode 1. The resistance of ions in the biological body to being taken as electrons into the electrode is the major component of the resistance Rs. The capacitance dl results from an electric double layer between the biological body and the biological electrode 1.

The artifact may be due to a high resistance (charge transfer resistance) Rs. Thus, lowering the resistance to electric charge conversion from ions to electrons is expected to decrease the occurrence of the artifact. More specifically, it is preferable to promote the interaction between chloride ions (Cl⁻) near the π electron conjugation of the graphene structure of carbon fibers on the surface of the biological body contact end 12 of the biological electrode 1.

Fig. 6 is a schematic view of charge transfer caused by contact between the biological electrode 1 according to an embodiment of the present invention and a biological body. As illustrated in Fig. 6, in a portion of the biological body contact end (a portion of the electrode E2) 12 of the biological electrode 1 according to an embodiment of the present invention from which the carbon fiber 101 is exposed, on the basis of the above thinking, part of the surface of the carbon fiber 101 is modified with a cationic functional group, more specifically a -NH₃⁺ group, for example. Thus, the surface of the biological body contact end (a portion of the electrode E2) 12 has a portion modified with a cationic functional group, and the cationic functional group is bonded to carbon of the graphene structure of the carbon fiber 101 in this portion.

As described above, the charge transfer substance on a biological body BB is chloride ions (Cl⁻). These chloride ions (Cl⁻) tend to electrically interact with the cationic functional group (-NH₃⁺ group) on the exposed surface of the carbon fiber 101 of the biological body contact end (a portion of the electrode E2) 12, which comes into contact with the biological body BB. This decreases polarization between the biological body BB and the carbon fiber 101 of the biological body contact end (a portion of the electrode 2) 12. Consequently, electric charges of chloride ions (Cl⁻) on the biological body BB can easily enter the π electron cloud of the graphene structure of the carbon fiber 101 of the biological body contact end (a portion of the electrode E2) 12. This also decreases temporal accumulation of electric charges and facilitates electric charge transfer between the biological body BB and the electrode E2 (the carbon fiber 101 of the biological body contact end 12). Thus, the biological electrode 1 according to an embodiment of the present invention enables stable measurement without using an electrolyte solution or an electrically conductive gel.

As illustrated in Fig. 6, in the biological electrode 1, the cationic functional group may include a cationic amino group (one example is a -NH₃⁺ group illustrated in Fig. 6). Part of the surface of the carbon material (the carbon fiber 101) is relatively easily modified with an amino group. An amino group can be converted into a cationic functional group by the addition of a proton. Thus, the use of a cationic amino group as a cationic functional group can easily increase the density of the cationic functional group on the surface of the carbon material (the carbon fiber 101) and can more stably reduce the effects of chloride ions (Cl⁻) of the biological body. The specific structure of the cationic amino group is not limited.

Nitrogen may be bonded to three hydrogens as in the-NH₃⁺ group illustrated in Fig. 6 or to an alkyl group R as in the groups represented by the general formula -NHR₂⁺. Other examples of the cationic functional group include cationic functional groups containing nitrogen, such as functional groups having an imidazoline structure (imidazolium cations) and functional groups having a pyridine structure (pyridinium cations), and cationic functional groups containing phosphorus, such as functional groups having a phosphine structure (quaternary phosphonium cations).

As a preferred example, a cationic functional group has formed a salt (ion pair, [-NH₃⁺] [Cl⁻]) with an anion (chloride ion) in Fig. 6. When the cationic functional group (-NH₃⁺ group) has not formed a salt ([-NH₃⁺] [Cl⁻]), an interaction between the cationic functional group and water produces hydroxide ions (OH⁻) around the cationic functional group. Thus, the solution around the carbon material (the carbon fiber 101) becomes alkaline, and hydroxide ions (OH⁻) are present around the cationic functional group (-NH₃⁺ group). Exchange between the hydroxide ions (OH⁻) and chloride ions (Cl⁻) from the biological body in this state changes the pH of the solution around the carbon material. In contrast, when the cationic functional group (-NH₃⁺ group) has previously formed a salt ([-NH₃⁺][Cl⁻]), exchange with chloride ions (Cl⁻) from the biological body can occur with no or little pH change of the solution around the carbon material.

In the biological electrode 1 on which at least part of the cationic functional group has formed a salt with an anion, the anion preferably includes chloride ions (Cl⁻). Chloride ions (Cl⁻) on a biological body interact with the carbon material (the carbon fiber 101) on the surface of the biological body contact end 12 of the electrode E2 and thereby transmit electric signals of biological information to the biological body contact end 12. Thus, previously forming an ion pair ([-NH₃⁺] [Cl⁻]) between a chloride ion (Cl⁻) and a cationic functional group bonded to carbon of the graphene structure of the carbon material (the carbon fiber 101) facilitates exchange between the chloride ions (Cl⁻) interacting with the cationic functional group and chloride ions (Cl⁻) from the biological body and can consistently decrease the possibility of chloride ions (Cl⁻) involved in polarization. Thus, in Fig. 6, the arrow starting from a chloride ion (Cl⁻) forming an ion pair with the cationic functional group (-NH₃⁺ group) represents the movement of an electron, and the arrow from a chloride ion (Cl⁻) to the chloride ion (Cl⁻) forming the ion pair represents ion exchange.

Thus, part of the surface of a portion of the electrode E2 made of a carbon material (the carbon fiber 101 of the biological body contact end (a portion of the electrode E2) 12 in Fig. 6) is modified with a cationic functional group, and the method of modification is not particularly limited. However, for example, modification with a -NH₃⁺ group, which is a cationic functional group, can be performed by the following method.

First, the biological electrode according to the comparative example is prepared (the shape and material are the same as those in the biological electrode 1 according to an embodiment of the present invention except that no cationic functional group is added, and the production method is also the same except that the surface treatment is not performed). This is used for measurement as the biological electrode according to the comparative example.

A portion of the biological electrode according to the comparative example corresponding to the biological body contact end 12 is immersed in concentrated nitric acid (60%). The liquid is maintained at approximately 25°C and is allowed to react for 24 hours. The biological electrode according to the comparative example is removed from the liquid after the reaction, is washed with pure water, and is dried. Part of the surface of a carbon fiber in the portion of the biological electrode according to the comparative example corresponding to the biological body contact end 12 is modified with a nitro group (-NO₂ group) through the reaction. This is tentatively referred to as a nitro-group-modified electrode. Part of the surface of the carbon fiber may also be modified with the nitro group (-NO₂ group) by immersion in a liquid containing concentrated nitric acid and acetic anhydride.

The portion of the nitro-group-modified electrode in which part of the surface of the carbon fiber is modified with the nitro group (-NO₂ group) (the portion corresponding to the biological body contact end 12) is then immersed in a liquid containing 100 mL of 28% aqueous ammonia and 1 g of sodium dithionate (Na₂S₂O₆) in a nitrogen atmosphere (actually in a nitrogen stream). The liquid is maintained at approximately 25°C and is allowed to react for 12 hours with stirring. The nitro-group-modified electrode is removed from the liquid after the reaction, is washed with pure water, and is dried. The nitro group (-NO₂ group) on the surface of the carbon fiber in the portion of the nitro-group-modified electrode corresponding to the biological body contact end 12 can be converted into an amino group (-NH₂ group) through the reaction. This is tentatively referred to as an amino-group-modified electrode. Approximately 0.5 g of sodium carbonate may be added to the liquid. The addition of sodium carbonate to increase alkalinity increases the reducing power of sodium dithionate and promotes amination.

The amino-group-modified electrode in which part of the surface of the carbon fiber is modified with the amino group (-NH₂ group) is immersed in hydrochloric acid (7% to 18%) for approximately 6 to 12 hours, is washed with pure water, and is dried. Thus, the biological electrode 1 can be produced in which part of the surface of the carbon fiber is modified with the cationic functional group (-NH₃⁺ group) that has formed a salt (ion pair) with a chloride ion (Cl⁻). The biological electrode 1 thus produced is hereinafter referred to as a "biological electrode according to an example".

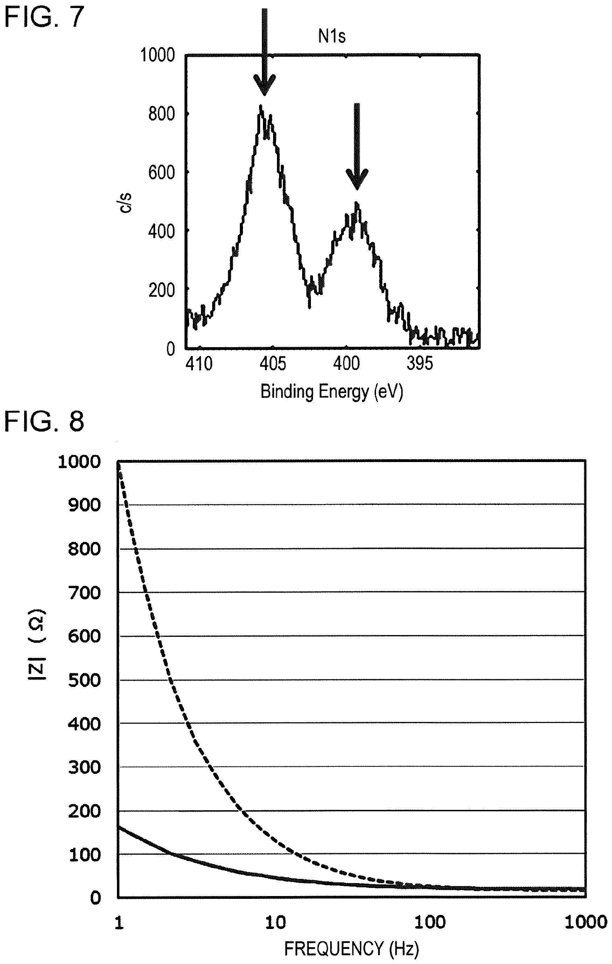

Fig. 7 is a graph of a result of X-ray photoelectron spectroscopy (XPS) measurement of the surface of a biological body contact end 12 of the biological electrode according to the example thus produced. Two peaks (approximately 400 eV and approximately 405 eV, indicated by the arrows in Fig. 7) are observed in the binding energy band of N1s. The 400 eV peak of these two peaks is derived from N bonded to H or C, and the 405 eV peak is derived from N bonded to O. Thus, the surface concentration (atomic percent) of N bonded to O can be calculated from the ratio of the area of the 400 eV peak to the total peak area and was approximately 1 atomic percent.

The produced biological electrode according to the example was immersed in 0.1 M saline and was subjected to impedance measurement. For comparison purposes, the biological electrode according to the comparative example was also tested. Fig. 8 shows the results. In Fig. 8, the solid line indicates the result of the biological electrode according to the example, and the broken line indicates the result of the biological electrode according to the comparative example. The horizontal axis represents frequency (Hz), and the vertical axis represents impedance (Ω).

In Fig. 8, the impedance of the biological electrode according to the example indicated by the solid line was 200 Ω or less even at a low frequency of approximately 1 Hz. In contrast, the impedance of the biological electrode according to the comparative example indicated by the broken line in Fig. 8 was approximately 1 kΩ at a low frequency of approximately 1 Hz. Thus, the graph of Fig. 8 shows that the above treatment of a portion of the biological electrode according to the comparative example corresponding to the biological body contact end 12 can decrease the impedance to approximately one-sixth.

The biological electrode according to the example and the biological electrode according to the comparative example were fixed to the forehead of a biological body (human), and an electroencephalogram was measured with an earlobe being a ground point. As a result, the biological electrode according to the example and the forehead had an impedance (1 Hz) of 98 kΩ, and the biological electrode according to the comparative example and the forehead had an impedance (1 Hz) of 141 kΩ. The biological electrode according to the example had a lower impedance (1 Hz).

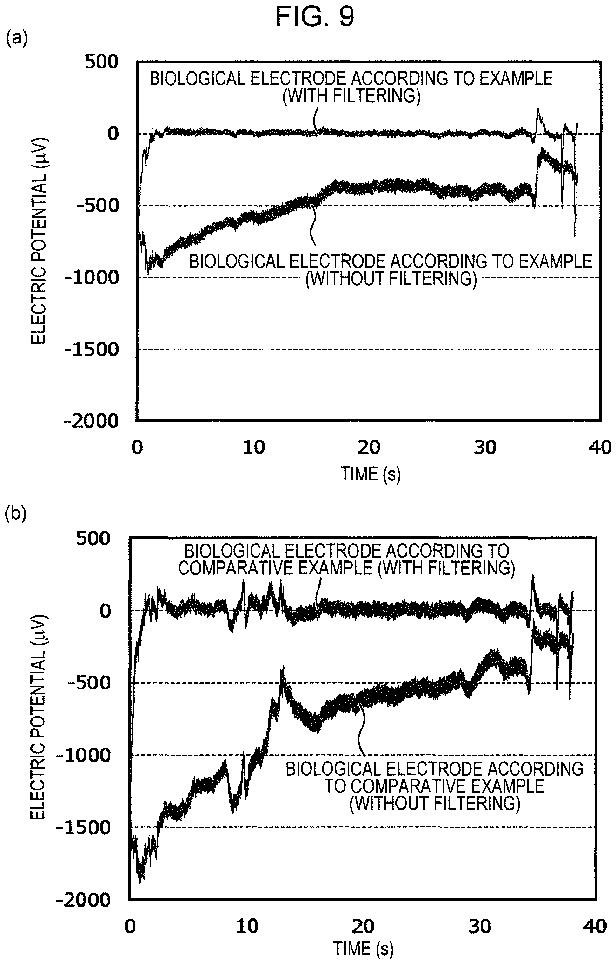

The variations in measured electric potential were also measured from the start of the measurement. Figs. 9(a) and 9(b) are graphs showing the variations in electric potential from the start of the measurement. As in Figs. 3 and 4, in both graphs, the horizontal axis represents the elapsed time (s), and the vertical axis represents the output electric potential (µV). As shown in Fig. 9(a), the biological electrode according to the example had no clear artifact and had an almost flat baseline after 0.5 Hz low-cut filtering. In contrast, as illustrated in Fig. 9(b), the biological electrode according to the comparative example had a clear artifact and did not have a flat baseline even after 0.5 Hz low-cut filtering. Thus, it was confirmed that the biological electrode according to the example could be used to stabilize the baseline.

A method of modification with a -NH₃⁺ group, which is a cationic functional group, in an ammonium carbamate solution is described below. This method (hereinafter referred to as a "carbamic acid method" for convenience) can be used for modification with a -NH₃⁺ group even when the materials of a biological electrode include a material that is prone to chemical damage, such as a resin, with less damage to such a material. The carbamic acid method includes an electrolytic oxidation step and a reduction step and preferably further includes a chlorination step.

First, an electrode for biological information measurement is prepared that includes an electrode, at least a portion of which is made of a carbon material, and a terminal electrically connected to the electrode, wherein at least part of the surface of the portion of the electrode made of the carbon material can come into contact with a biological body.

In the electrolytic oxidation step, the surface of the electrode for biological information measurement is brought into contact with an ammonium carbamate solution. The solvent for the ammonium carbamate solution is water, for example. The concentration of ammonium carbamate in the ammonium carbamate solution is not particularly limited, provided that the following electrolysis can be performed. To more stably perform electrolysis, the concentration of ammonium carbamate preferably ranges from 0.01 to 1 M. To more stably perform electrolysis, the ammonium carbamate solution may contain an electrolyte, such as sodium chloride. The ammonium carbamate solution may preferably have a pH in the range of 8 to 12. The ammonium carbamate solution may contain a buffer, such as a phosphate, provided that electrolysis is not affected.

While the surface of the electrode for biological information measurement is in contact with the ammonium carbamate solution, the surface is anodically electrolyzed. The electric potential of the anodic electrolysis is preferably equal to or higher than the oxidation potential (1 V) of ammonium carbamate and lower than the electrolysis potential of the solvent. When water is used as a solvent, the anodic electrolysis is performed at a voltage of less than approximately 1.6 V. A non-limiting example of the electric potential of the anodic electrolysis ranges from 1.0 to 1.4 V, preferably 1.1 to 1.3 V. Thus, the carbon material can be modified with the amino group by the anodic electrolysis.

The temperature of the ammonium carbamate solution in the anodic electrolysis is not particularly limited. To perform uniform treatment, the temperature may preferably range from 10°C to 60°C. The electric current density in the anodic electrolysis is not particularly limited. Basically, the amount of amino group modifying the carbon material increases with the anodic electrolysis time. The anodic electrolysis time is determined in consideration of the amount of amino group to modify the carbon material and productivity. A non-limiting example of the anodic electrolysis time ranges from 10 minutes to 10 hours and may preferably range from 30 minutes to 5 hours.

In the reduction step, the surface of the electrode for biological information measurement oxidized in the electrolytic oxidation step is brought into contact with a solution containing ammonia, sodium dithionate, and sodium carbonate (hereinafter also referred to as a "reduction solution") to reduce the portion oxidized by the anodic oxidation. Through this reduction, a portion of a carbon fiber oxidized simultaneously with amination is reduced, and the amino group modifying the carbon material on the surface of the electrode for biological information measurement functions further effectively.

The reduction solution is produced, for example, by adding 1% by mass sodium dithionate and 0.5% by mass sodium carbonate to 28% aqueous ammonia. The surface of the electrode for biological information measurement may be brought into contact with the reduction solution by any method. The electrode for biological information measurement may be immersed in the reduction solution, or the reduction solution may be sprayed on the electrode for biological information measurement. The contact time between the surface of the electrode for biological information measurement and the reduction solution is appropriately determined according to the other conditions, such as the temperature of the reduction solution. A non-limiting example of the contact time is approximately 12 hours when the reduction solution is at room temperature (25°C).

In the chlorination step, the surface of the electrode for biological information measurement reduced in the reduction step is brought into contact with an acidic liquid containing chloride ions. The acidic liquid is approximately 10% hydrochloric acid, for example. Thus, the carbon material on the surface of the electrode for biological information measurement is modified with the cationic functional group (-NH₃⁺ group).

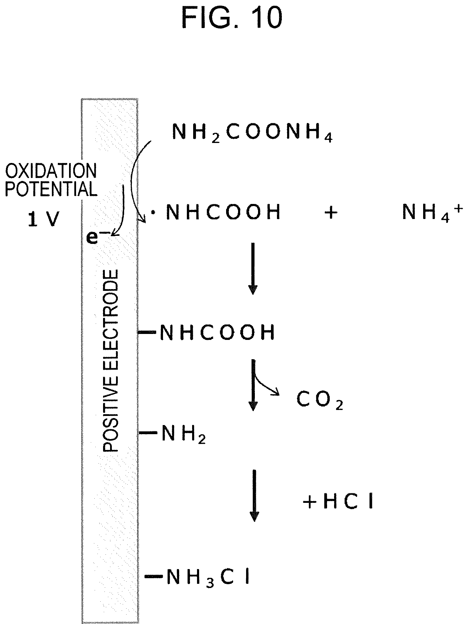

Fig. 10 schematically illustrates the above process. Amination by electrolytic oxidation utilizes the decomposition of ammonium carbamate, which is unstable and easily decomposes into ammonia and carbon dioxide. Ammonium carbamate is oxidatively decomposed on the positive electrode (anodic electrolysis), and carbamic acid is directly covalently bonded to a cross section of a carbon fiber. Decarboxylation of the carbamic acid results in an amino group covalently bonded to the carbon fiber.

A method for producing an electrode for biological information measurement (the biological electrode 1) by the carbamic acid method is described in detail in the following example.

### (1) Type of Carbon Fiber Electrode and Comparison Electrode

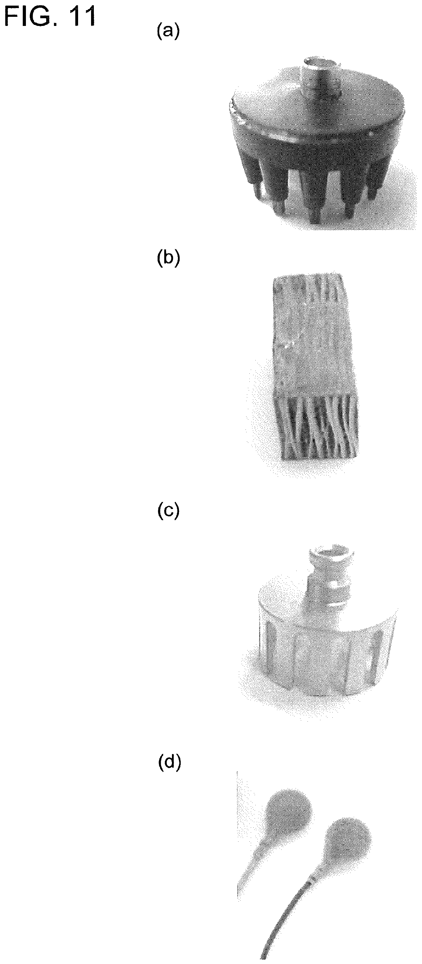

(1-1) Carbon Fiber Focusing Electrode
   A prepreg manufactured by Nippon Graphite Fiber Co., Ltd. was cut, was lightly dissolved in acetone, and was formed in the cylindrical shape through a circular dispenser tip. While being hanged, the formed product was cured at 130°C for 2 hours. The formed product was cut with nippers to an appropriate length, and 13 pieces of the formed product were attached to a rubber case to fabricate an electrode in the shape as shown in Fig. 11(a).
(1-2) A 4 mm x 5 mm x 10 mm CC composite block (manufactured by Toyo Tanso Co., Ltd., Fig. 11(b)) was prepared.
(1-3) A "Parthenon" manufactured by Miyuki Giken Co., Ltd. (a three-dimensional electrode made of gold-plated brass, Fig. 11(c)) and a gold-plated dish shaped electrode manufactured by Miyuki Giken Co., Ltd. (Fig. 11(d)) were prepared as comparison electrodes.
(1-4) A ten20 manufactured by Weaver and Company was prepared as a conductive paste for electroencephalography electrode.

### (2) Carbamic acid Method

### (2-1) Electrolytic Oxidation Step

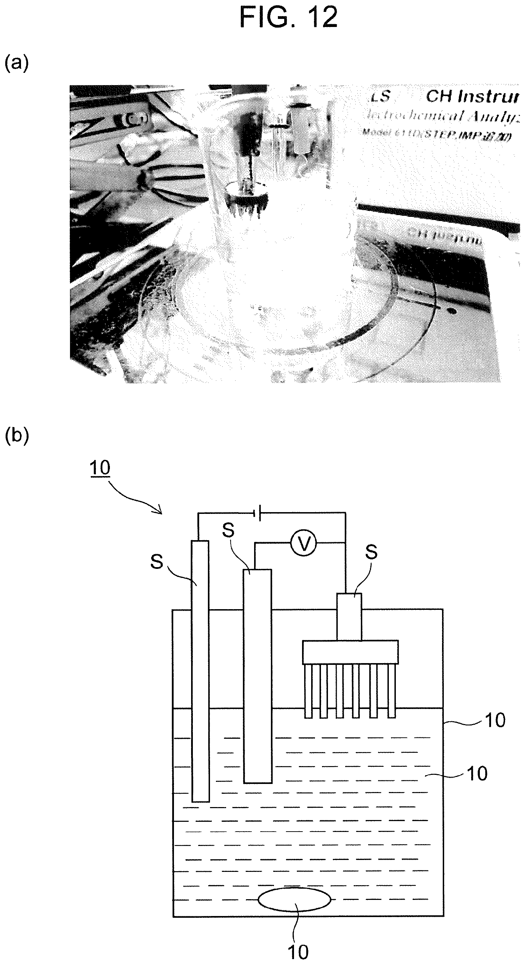

Fig. 12(a) is a photograph of an electrolytic treatment apparatus (electrolyzer). Fig. 12(b) is a schematic view of the structure of an electrolytic treatment apparatus (electrolyzer). As shown in Figs. 12(a) and 12(b), 0.1 M aqueous ammonium carbamate 103 was poured into a vessel 101 containing a stirrer chip 102 in an electrolyzer 100, and a sample SP of a carbon fiber focusing electrode, a reference electrode S1, and an auxiliary electrode S2 were immersed in the aqueous ammonium carbamate 103. A voltage was applied from the auxiliary electrode S2 for 2 hours such that the voltage was 1.2 V between the reference electrode S1 and the sample SP. The reference electrode S1 was a calomel electrode (Hg/Hg₂Cl₂), and the auxiliary electrode S2 was a Pt electrode.

### (2-2) Reduction Step

1% by mass sodium dithionate as a reducing agent and 0.5% by mass sodium carbonate were added to aqueous ammonia (28%) to adjust a reduction solution. The sample SP subjected to the electrolytic oxidation step and washed with water was immersed in the reduction solution at room temperature (25°C) for 12 hours.

### (2-3) Chlorination Step

The sample SP subjected to the electrolytic step and washed with water was immersed in approximately 10% hydrochloric acid (room temperature (25°C)) for 5 hours. The immersed sample SP was washed with water and was dried to prepare an electrode for biological information measurement (the biological electrode 1).

### (3) Measurement and Evaluation

### (3-1) Anodic Electrolysis Time and N Concentration

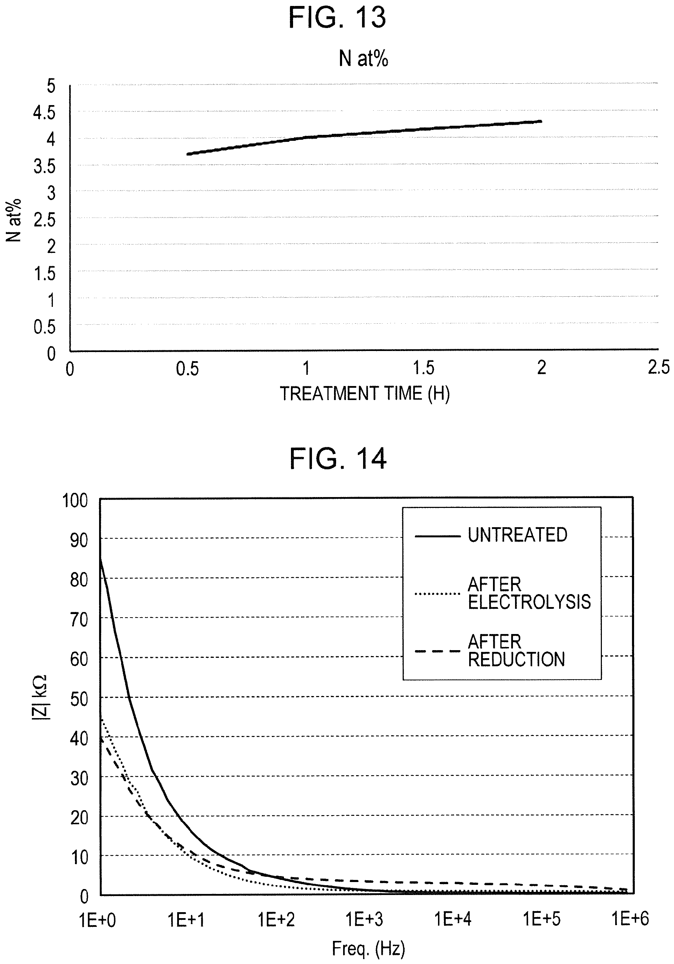

The CC composite shown in Fig. 11(b) was subjected to electrolytic oxidation at 1.2 V in 0.1 M aqueous ammonium carbamate in the electrolytic oxidation step. After the electrolytic oxidation step, a cross section of the tip of the electrode was observed by electron spectroscopy for chemical analysis (ESCA). Fig. 13 shows the result. As shown in Fig. 13, approximately 3.5 at% N (derived from NH₂) was detected at a treatment time of 0.5 hours. The amount of N increased gradually with the treatment time and was approximately 4.3 at% at a treatment time of 2 hours.

### (3-2) Measurement of Impedance

A sample SP of a carbon fiber focusing electrode before the electrolytic oxidation step with an application time of 2 hours (untreated, the solid line in Fig. 14), the sample SP after the electrolytic oxidation step (after electrolysis, the dotted line in Fig. 14), and the sample SP after the electrolytic oxidation step and the reduction step (after reduction, the broken line in Fig. 14) were measured for the impedance with respect to 0.1 M saline. Fig. 14 shows the results. As shown in Fig. 14, the electrolytic oxidation step decreased the impedance by almost half compared with the untreated sample, and the additional reduction step decreased the impedance by approximately 10% compared with the sample after only the electrolytic oxidation step.

### (3-3) Measurement of Electroencephalogram

An electroencephalogram was measured with an electroencephalography system equipped with an active electrode (Polymate AP108) manufactured by Miyuki Giken Co., Ltd. The measuring point was the forehead. The three-dimensional comparison electrode and the carbon fiber focusing electrode subjected to the electrolytic oxidation step and the reduction step were symmetrically attached to the forehead in the measurement with the electroencephalography system. The gold-plated dish shaped electrode coated with ten20 was attached to an earlobe as a reference. The impedance with respect to the forehead was 102 kΩ in the comparison electrode and 64 kΩ in the carbon fiber focusing electrode.

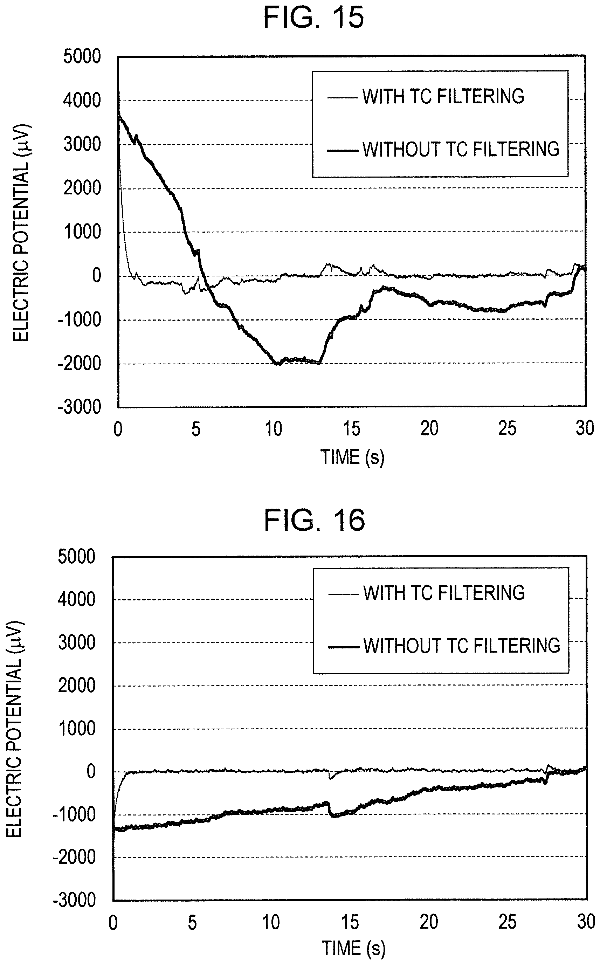

Fig. 15 (comparison electrode) and Fig. 16 (carbon fiber focusing electrode) show the results (with TC filtering, thin lines) of HFF filtering (cutting 60 Hz or more), TC filtering (time constant 0.3 s, cutting 0.5 Hz or less), and Notch filtering (50 Hz) and the results (without TC filtering, thick lines) of the potential difference from the reference without TC filtering of the measurement results (only with HFF and Notch filtering).

The electric potential change of the carbon fiber focusing electrode in Fig. 16 is smaller than the electric potential change of the comparison electrode in Fig. 15. In particular, without TC filtering (the thick lines in Figs. 15 and 16), the electric potential of the comparison electrode (Fig. 15) varies greatly with respect to the reference, but the electric potential of the carbon fiber focusing electrode (Fig. 16) gradually approaches the electric potential of the reference. Thus, it is confirmed that when the carbon fiber focusing electrode is used, additional TC filtering enables stable measurement of electric potential.

As described above, in an electrode for biological information measurement (the biological electrode 1) according to an embodiment of the present invention, the biological body contact end 12, which is a portion of the electrode E2 made of a carbon material, has a surface that can come into contact with a biological body, and part of the surface is modified with a cationic functional group. Thus, electric charges of chloride ions on the biological body can easily enter the π electron cloud of carbon on the electrode E and decreases polarization between the biological body and the electrode E2. This also decreases temporal accumulation of electric charges and facilitates electric charge transfer between the biological body and the electrode E2. This enables stable measurement without using an electrolyte solution or an electrically conductive gel.

### Reference Signs List

1 biological electrode
E2 electrode
10 pins
12 end (biological body contact end)
20 base
30 terminal
200 biological electrode according to related art (gold electrode)
201 pins
202 sphere
203 base
204 terminal
101 carbon fiber
BB biological body

## Claims

1. An electrode for biological information measurement comprising:
an electrode, at least a portion of which is made of a carbon material; and
a terminal electrically connected to the electrode,
wherein at least part of a surface of the portion of the electrode made of the carbon material can come into contact with a biological body, and
part of the surface is modified with a cationic functional group.

2. The electrode for biological information measurement according to Claim 1, wherein the cationic functional group includes a cationic amino group.

3. The electrode for biological information measurement according to Claim 1 or 2, wherein at least part of the cationic functional group has formed a salt with an anion.

4. The electrode for biological information measurement according to Claim 3, wherein the anion includes chloride ions.

5. A method for producing an electrode for biological information measurement,
the electrode for biological information measurement including an electrode, at least a portion of which is made of a carbon material, and a terminal electrically connected to the electrode, wherein at least part of a surface of the portion of the electrode made of the carbon material can come into contact with a biological body,
the method comprising:
an electrolytic oxidation step of anodically electrolyzing the surface while the surface is in contact with an ammonium carbamate solution; and
a reduction step of bringing the surface oxidized in the electrolytic oxidation step into contact with a solution containing ammonia, sodium dithionate, and sodium carbonate to reduce the surface.

6. The method for producing an electrode for biological information measurement according to Claim 5, further comprising a chlorination step of bringing the surface reduced in the reduction step into contact with an acidic liquid containing chloride ions.
